(19) Europäisches Patentamt
European Patent Office
Office européen des brevets

(11) Publication number: 0 046 326 A2

(12) # EUROPEAN PATENT APPLICATION

(21) Application number: 81200918.1

(22) Date of filing: 18.08.81

(51) Int. Cl.³: A 61 K 7/00, A 61 K 35/20

(30) Priority: 19.08.80 US 179391

(43) Date of publication of application: 24.02.82 Bulletin 82/8

(84) Designated Contracting States: AT BE CH DE FR GB IT LI NL SE

(71) Applicant: STAUFFER CHEMICAL COMPANY, Westport Connecticut 06880 (US)

(72) Inventor: Staples, Lorna Carol, 66 Bilton Street, Teaneck New Jersey 07666 (US)

(74) Representative: Urbanus, Henricus Maria, Ir. et al, c/o Vereenigde Octrooibureaux Nieuwe Parklaan 107, NL-2587 BP 's-Gravenhage (NL)

(54) **Whey protein containing cosmetic formulations, process of preparing the same and a method for setting hair by using a non-hydrolyzed whey product.**

(57) A cosmetic formulation for treating skin and hair having incorporated therein a substantially non-hydrolyzed whey protein-containing product. A method of preparing the same and a method for setting hair by using said cosmetic formulation. The whey products provide substantive results without the expense of hydrolysis.

EP 0 046 326 A2

WHEY PROTEIN CONTAINING COSMETIC FORMULATIONS, PROCESS OF PREPARING THE SAME AND A METHOD FOR SETTING HAIR BY USING A NON-HYDROLYZED WHEY PRODUCT

The invention relates to cosmetic compositions which are applied to keratinous material such as skin or hair.

Many attempts have been made in the past to provide compositions for maintaining or improving the condition of skin or hair. Protein has been applied to skin and hair for thousands of years. Casein, in the form of milk, has been used extensively as a beautifier and more recently, it has been recommended for use in toilet soaps. U.S. Patent No. 3,548,056 describes the inclusion of partially degraded proteins having a gel strength of 0 Bloom grams in detergent compositions and lotions for application to the skin such as dishwashing liquids. U.S. Patent No. 3,898,186 discloses liquid detergent compositions containing non-ionic surfactants and unmodified gelatins having a molecular weight of at least 12,500, an isoelectric point between pH 4.5 and 9.2 and a gel strength between 25 and 300 Bloom grams.

Of all the various proteins and their derivatives which have been used in cosmetics, only hydrolyzed protein and particularly, hydrolyzed animal protein was considered the generally acceptable protein for use in cosmetics. Some native proteins are in a form not easily used in cosmetics. Some natural proteins are water insoluble. Some water soluble proteins are pH sensitive and precipitate at their isoelectric point. Native protein can be easily denatured by heat resulting in a coagulated material. Denatured proteins have poor solubility and are unsuitable for use in cosmetics. Some native proteins are biologically active and, therefore, sensitizers. Hydrolyzed protein lacks the native structure of the protein as the protein is split into lower molecular weight peptides. This allows the protein to more easily associate with the hair and skin. Acid, alkaline or enzyme hydrolysis is used to prepare proteins for cosmetics. In the first two, the protein is broken down in a nonspecific manner whereas in the latter case the enzyme is specific and the molecular weight which determines protein absorption by the hair and skin can be precisely controlled. The protein can also be chemically modified to provide suitable characteristics for various cosmetic uses.

Of the numerous protein containing cosmetics, the majority contain hydrolyzed animal protein, i.e., hydrolyzed collagen or gelatin. Collagen is an abundunt raw material which is easily hydrolyzed to a low Bloom product having a low molecular weight. One company reports that a molecular weight of about 1,000 provides the highest substantivity to damaged hair (Protein Facts, Inolex, page 9). While the prior art has, in general, stated that only low molecular weight protein is useful, a product based on oats has been introduced in the market stating:

> "To our knowledge this is the only unprocessed, naturally-occurring protein that has been available to the cosmetics industry. This means that for the first time, whole natural proteins, and the inevitable dermatological attributes, can be formulated for skin products". (A New Natural Ingredient For Cosmetic Formulators, Drug and Cosmetic Industry, September 1973).

Therefore, there is a need to offer the cosmetic chemist another new protein system which will consist of native protein and overcome the known disadvantages of native protein in cosmetics.

## SUMMARY OF THE INVENTION

In accordance with the invention, there is provided a new group of protein-containing cosmetics having incorporated therein a substantially non-hydrolyzed whey protein-containing product. These cosmetics can be prepared with a protein product which does not need to be hydrolyzed eliminating the expense of that procedure. The whey products are able to provide substantive benefits even though they are not hydrolyzed.

## DETAILED DESCRIPTION OF THE INVENTION

### WHEY PRODUCTS

The whey products which are used in the invention are substantially non-hydrolyzed whey protein-containing products including whey, delactosed whey, demineralized whey, delactosed demineralized whey, whey protein concentrates prepared by physical or chemical concentration techniques, the precipitate obtained by neutralizing acid whey or a calcium enriched whey, the material remaining after separating the precipitate, the byproducts resulting from the preparation of whey protein concentrates, and mixtures thereof.

The whey used in preparing the materials of the invention can be acid whey, sweet whey, or mixtures thereof. More particularly, the whey can be cottage cheese whey, casein whey, cheddar cheese whey, mozzarella cheese whey, Swiss cheese whey and the like or mixtures thereof. No significant difference is known in preparing cosmetic products prepared from acid or sweet whey though products prepared substantially (at least about 75%) from acid whey are preferred.

The whey, whether acid, sweet or blends thereof, can be used dry or in liquid form (raw or concentrated). The protein of the whey can be concentrated using various physical techniques including delactosing by crystallization, molecular sieve fractionation (Reissue Patent 27,806), Ultrafiltration (Horton, B.S. et al., Food Technology, Vol. 26, p. 30, 1972), reverse osmosis ("Fractionation and Concentration of Whey by Reverse Osmosis" by Marshall, P.G., Dunkley, W.L. and Lowe, E., Food Technology, Vol. 22(a), pp. 969-1968), dialysis and electrodialysis (Desalting By Electrodialysis, by Friedlander, H.Z., and Rickles, R.W., Chem. Engineering, May 23, 1966, p. 153) and the like. The preferred technique is ultrafiltration (discussed more fully hereinafter). The protein can also be chemically concentrated by the use of inorganic phosphates (2,377,624 and 4,043,990) or sodium lauryl sulfate (4,029,825 and 4,058,510). A whey protein concentrate is defined as a product which has been treated in a way to increase the protein content of the dry product above its normal content of about 11% and preferably, to an increased level of at least about 25% protein based on total Kjeldahl nitrogen. Upper limits of up to 95% of the dry material can be obtained. Preferably, the whey protein concentration ranges from about 40% to about 60% by weight of the dry material.

## WHEY BYPRODUCTS

Included in the class of whey products used in the invention are certain whey byproducts in addition to the whey products described above. These byproducts are protein-containing residues derived from either acid or sweet cheese whey which has been processed to remove a part of the protein content thereof. The preferred whey byproducts include the second fraction obtained from the molecular sieve separation of cheese whey as described in U.S. Reissue 27,806, the permeate obtained from the ultrafiltration concentration of protein from whey, and delactosed permeate in either liquid or dried form.

The low molecular weight second fraction is the material obtained by passing a partially delactosed cheese whey mother liquor through a bed of molecular sieve resin in accordance with Reissue Patent No. 27,806 and recovering, for the purposes of this invention, the low molecular weight second fraction containing mainly lactose, ash and residual protein. The partially delactosed whey mother liquor is obtained by concentrating raw cheese whey by conventional means to a solids concentration of about 60%, reducing the temperature of the concentrate to induce lactose crystallization and thereafter separating crystalline lactose from the liquid by conventional means.

If desired, the whey used in preparing the whey protein concentrates and the byproducts can be pretreated to clarify the whey using processes such as centrifugation and processes such as illustrated in U.S. Patent No. 3,560,219. In accordance with this patent, lipid is removed as a precipitate from whey by treating the whey solution with calcium ion at approximately a neutral pH. Acid whey or blends of acid and sweet whey (at least about 40% acid whey on a weight basis based on the weight of the whey or blend) can be clarified by elevating the pH to neutral as disclosed in U.S. Patent No. 4,036,999. The yield of precipitate is maximized by heating. A temperature of 55-60°C. is suggested. The latter can also be admixed with calcium ion and heat treated. The disclosure of these two patents is incorporated herein by reference.

The precipitate from the processes of U.S. Patent Nos. 3,560,219 and 4,036,999 after pasteurization can be used as whey products in the invention. These products are identified in the specification and claims as the precipitates obtained by neutralizing acid whey or a calcium enriched whey, preferably, sweet whey.

The liquor remaining after the separation of the precipitates is high in protein, lactose and ash. This material can be used directly in cosmetics or the whey protein can be concentrated by removing lactose and/or ash. The liquor is preferably processed by physical concentration techniques such as outlined hereinbefore, preferably gel filtration or ultrafiltration and more preferably, ultrafiltration. Preferably, the whey stream used in the gel filtration or ultrafiltration fractionation of whey is clarified prior to delactosing.

Ultrafiltration membranes are utilized to separate the high molecular weight fraction of the whey (protein) from the liquid and low molecular materials, i.e., low molecular weight protein, lactose and ash in the whey solution. The protein enriched solution is retained on the membrane and it is called the retentate. The water and low molecular weight fraction passes through the membrane and is called the permeate.

In an illustrative method for ultrafiltering cheese whey, a whey protein concentrate containing from about 40% to about 60% and preferably 50% ± 5% whey protein on a dry basis is prepared by neutralizing acid whey to a pH of 6.5 with caustic. After storage at a temperature of about 57°C., the pH is then adjusted to 7.2 and any solids or precipitates are removed by centrifugal clarification. The clarified liquor is then pasteurized and fed to an ultrafiltration membrane unit. The retentate is condensed and spray dried. The liquid permeate can be dried as is or concentrated and/or delactosed by concentration and cooling to effect a precipitation of a lactose. The drying of the permeate or delactosed permeate (DLP) can be facilitated by the use of drying adjuvants such as starch and alkali metal caseinates.

Protein products of 35% by weight (dry basis) or more whey protein are efficiently prepared by ultrafiltration. One of the more preferred products prepared by this process generally comprises from about 40% to about 60% whey protein based on Total Kjeldahl Nitrogen, 10-30% lactose, 3-15% ash and 0.1-4% fat, said percentages being by weight based on the weight of the protein product (on a dry solids basis).

The dried permeate from an ultrafiltration has a general composition of:

Lactose, %---------------------70-80
Ash, %-------------------------10-15
Protein, (N x 6.38), %--------- 4-8
Fat, %------------------------- less than 1
Moisture ---------------------- less than 5
pH ---------------------------- 6-7

After removing a portion of the lactose by crystallization procedures from a solution, the delactosed permeate contains about 40-45% lactose, about 25-35% ash and about 8-12% protein (TKN x 6.38) on a dry basis.

It is preferred to utilize the delactosed permeate with a drying adjuvant such as casein or starch. A particularly preferred combination is delactosed permeate and from about 20% to about 75% starch. The starch is preferably a thin-boiling starch made by controlled acid hydrolysis of starch in the granular state at about 52°C. using sulfuric or hydrochloric acid as catalyst. Cold water stability is dependent on the amount of amylose in the starch.

The preferred starch is a low amylose modified tapioca starch which is thin boiling and has little tendency to gel on cooking. For purposes of the present invention, products with a D.E. (Dextrose Equivalents) of about 30 and preferably about 15 or less are useful.

The blend of the deproteinized whey byproduct solution and starch can be codried by any known means. It has been found that a particularly useful product is obtained using a ratio of delactosed permeate to tapioca dextrin of about 2:1 (by weight based on total solids).

It is particularly preferred to utilize, as the whey protein concentrate, a product which has been treated to reduce the thermal gelation temperature such as by the treatment of the protein with sulfide or preferably, in accordance with the method disclosed in Serial No. 95,684, filed November 19, 1979, the disclosure of which is incorporated herein by reference. The process as described in this copending application comprises cooling a heated alkaline protein containing solution having a total protein content of less than about 20% by weight of the solution wherein the pH is within the range of from about 8 to about 10 from a temperature within the range of from about 50°C. to the gelation temperature of the protein to a temperature sufficiently low and within a sufficient amount of time after the solution reaches its maximum temperature level to prevent any substantial further change in the protein structure. The pH of the material is preferably reduced to neutral (6-8) simultaneously with, or subsequent to cooling.

The whey protein which can be treated by this process is that protein which is substantially soluble at an alkaline pH of between 8 and 10. It is preferable that the protein be at least 50% by weight soluble and preferably at least 75% and more preferably at least 100% soluble at the alkaline pH of the process. The major portion of protein (at least 50% by weight protein) for use in the aforementioned process is preferably derived from any whey protein concentrate though the material can contain minor amounts of protein from other sources including dairy such as milk, whey byproducts and whey, vegetable such as soy, cottonseed, peanut and the like vegetables, soluble meat proteins such as those obtained from red meat, poultry and fish as well as egg and blood albumens. Preferably, the whey protein material which is treated is a whey protein concentrate provided by the processes previously discussed. The whey protein concentrate should contain at least 25% by weight (dry basis) and preferably from about 40% to about 60% protein based on Total Kjeldahl Nitrogen. Processing which substantially denatures the whey protein such as high heat, strong chemicals and electrodialysis under extreme conditions should be avoided. It has been found that the most effective results are obtained using an ultrafiltered acid whey concentrate containing from about 40% to about 60% by weight (dry basis) protein. The process for obtaining such a product has been previously described as well as its composition.

The whey protein containing solution to be treated by the process disclosed in copending application Serial No. 95,684 does not require a minimum amount of protein for effective treatment. It is preferred to use a whey protein solution having from about 0.5% to about 20% by weight total protein in solution. The protein containing solution can be obtained by the use of an existing whey protein concentrate solution or by rehydrating a dried product in water. The pH is then adjusted to a range of from about 8 to about 10, preferably for whey proteins from about 9 to about 10 with most effective results being achieved at about 9.5. Any food grade alkalizing agent such as sodium or potassium hydroxide and preferably sodium hydroxide can be used. Other methods of elevating the pH such as by the use of an anionic/cationic exchange resin can be used. Sufficient agitation is utilized to avoid localization of high pH.

The alkaline whey solution is then heated to a temperature within the range of from about 50°C. to about 80°C., the temperature being elevated as fast as possible without causing protein insolubilization. The maximum heating time is preferably not over a maximum of about 1 hour and more preferably less than about 30 minutes, and most preferably less than about 15 minutes, depending on the temperature of processing. High temperature, short time pasteurization processing equipment can be effectively used for heating and cooling. After alkalization and heating, the product is cooled to a temperature within the range of from about 2°C. to about 30°C. within at least 60 minutes and preferably within about 15 minutes after reaching the elevated temperature at a rate sufficient to prevent any further substantial change in the protein structure and preferably to room temperature. Cooling must be initiated after the protein has been treated and before substantial gelation has occurred. The higher temperature achieved, the greater the rate required for the cooling. Subsequent to cooling, the pH is adjusted to a pH below 8 and preferably within the range of from about 6 to about 8 and more preferably from about 6.5 to about 7.5 with any food grade acid. Sufficient agitation should be utilized to avoid localized conditions of acid build-up. The acid can be added during the cooling step if desired.

The neutralized product can be used as is, concentrated and/or dried. Effective material handling conditions should be observed to avoid spoilage and contamination of the product depending on its physical form.

The product prepared by the process of Serial No. 95,684 or whey protein concentrates in general can be used in amounts of at least 50% by weight and more preferably at least 75% and most preferably 100% whey protein concentrate including modified whey protein concentrate with the remaining protein comprising blends of other proteins modified by the process of 95,684 or non-modified proteins such as milk, alkali metal caseinates, unmodified whey proteins including dry whey, delactosed whey, delactosed demineralized whey, the dried permeate and delactosed permeate resulting from the ultrafiltration of whey, the precipitate prepared by neutralizing acid whey as disclosed in U.S. Patent No. 4,036,999 and the precipitate prepared by adding calcium ion to sweet whey followed by neutralization as disclosed in U.S. Patent No. 3,560,219 as well as the dried mother liquor remaining after separation of these precipitates, vegetable proteins such as soy proteins and soluble protein such as egg albumen and blood albumen whole egg and soy yolk. The use of these materials is dependent on the gel formation and hence, the water solubility thereof. A minor amount of hydrolyzed protein can also be blended with the whey product if desired. Hydrolyzed protein is exemplified by hydrolyzed casein, hydrolyzed collagen, or any other such protein normally used in cosmetics, such as oat protein.

Illustrative of these blends is the blends of from about 25% to about 75%, preferably from about 60% to about 70% of the precipitate prepared by the processes of 3,560,219 or 4,036,999 and from about 75% to about 25% and preferably from about 40% to about 30% of a whey protein concentrate. Preferably, the whey protein concentrate is substantially denatured (at least 40%). The percentages are by weight based on the dry solids weight of the blend.

These blends of protein can be made by dry blending or codrying the liquid blend. When using a modified whey protein concentrate (Serial No. 95,684), only a small amount of additional unmodified protein, i.e., less than 25% is recommended to avoid diluting the effects of the modified protein.

While the product can be dried effectively by itself, it is also contemplated to codry the products with drying agents and other functional ingredients, i.e., gums, starch, stabilizers, bulking agents, emulsifiers and the like materials.

The whey products can be used to effectively enhance the characteristics of various cosmetics which are principally used on the hair and skin. Hair products can be illustrated by shampoo, hair groom, cream rinse, conditioner, setting lotion, permanent wave, hair straightener, dyes and colorings, hair lighteners including bleaches and dye removers and the like. Products for treating the skin include cleansing formulations such as body lotion, cold cream, cleansing cream or lotion, waterless hand cleaners, emollient formulations, therapeutic formulations including hormone formulations, and topical pharmaceuticals, softening formulations and the like. Other cosmetics include foundations for makeup, beauty masks, makeup including rouge, lipstick and eye makeup, shaving preparations, depilatories, suntan and sun screening preparations, dusting powders, bath preparations, soaps and detergents, including dishwashing detergents and the like. Any product can be in any convenient form, such as creams, lotions, cakes, powders, liquids, emulsions, aerosols, pastes and gels, sticks and the like. Because of the pure nature of the whey products, it is expected that they can also be effectively used in baby cosmetics. While not generally included in the term "cosmetics", products which are intended for softening the hands such as liquid dishwashing detergents and materials such as waterless hand cleaners, are intended to be included because of the cosmetic effect relating to the cleansing and softening of the skin.

Formulations for these types of materials are well known in the industry. Illustrative specific formulations for the products noted with the exception of dishwashing liquid and waterless hand cleaner can be seen in the text entitled, Cosmetics: Science and Technology, (2nd Edition), by M.S. Balsam et al., copyright 1972 by John Wiley and Sons in two volumes. This material is incorporated herein by reference.

The whey product is generally used in a cosmetic formulation in an amount sufficient to provide effective results. Generally, the amount for cosmetic usage ranges from about 0.5% to about 10% preferably 0.75% to about 5% and more preferably from about 0.75% to about 3% by weight based on the total weight of the cosmetic formulation. These amounts are given as general ranges for all cosmetics though the amounts used for specific cosmetics may vary slightly. Those variances are easily determined by one of ordinary skill in the art. The effectiveness of the whey product is generally dependent on the protein content and the type of protein. The whey product has at least about 3% and preferably at least about 10% protein. Use of amounts in excess of that needed for effective results are not recommended due to the cost of the whey product.

The cosmetic formulation containing the whey product can be easily prepared by using the procedure generally used in manufacture of the cosmetic and adding the protein at the end of the manufacturing procedure. The whey products are easily formulated with other cosmetic ingredients. They can be added as powders or presolubilized as desired. Since the protein is substantially undenatured, the protein is heat sensitive and the addition of the protein at too high a temperature (above about 50°C.) could coagulate the protein. Since most recipes add a fragrance at the end of the manufacturing procedure at about 50°C., it is convenient to add the protein at this point. If the protein is solubilized first before addition, a little more latitude on temperature exists. However, elevated temperatures (above 80°C.) are to be avoided. The whey product treated to lower its thermogelation temperature is especially sensitive to temperature. Temperatures of above about 65°C. for the use of this product are to be avoided.

Inasmuch as the whey product is a natural material and contains bacteria even after pasteurization, it is necessary that the formulations be kept refrigerated or be sufficiently acidic or alkaline to retard bacterial growth or include a preservation agent to avoid extensive bacterial growth. Alkyl parabens are effective preservation agents. In addition, it is contemplated that the cosmetic using the whey protein product contains the usual oils, emulsifiers, stabilizers, dyes, pigments, and other ingredients as would be found in such cosmetics in the amounts normally utilized.

Some of the benefits which can be achieved using the whey product of the invention include smooth after-feel, enhanced emulsion stability, increased apparent viscosity, reduced greasy feel of oils, soothing effect on irritated skin, improved pigment dispersion, increased adhesion of agents such as sun screens or powder to the skin, absorbs both water and oil, and addition of natural protein to skin and hair. The whey product can also act as a compression aid in pressed powders. While not all whey products can provide these results to all cosmetics, each whey product can provide some of these benefits to a cosmetic.

Since some of the whey products have the ability to thermogel, it is contemplated that the whey product and particularly the modified whey protein concentrate prepared by Serial No. 95,684 be used in a hair setting lotion to provide the set to the hair. It is contemplated that the protein can be caused to gel under elevated temperatures such as that achieved using a hair dryer.

The present invention is further illustrated in the examples which follow.

## EXAMPLE 1

A shampoo formulation was prepared using four whey products*, i.e., 1. whey protein concentrate (50% protein), 2. a blend of delactosed permeate and tapioca dextrin at a dry solids weight ratio of 2:1, 3. a blend of 65% of the precipitate prepared by neutralizing acid whey according to U.S. Patent No. 4,036,999 and 35% denatured whey protein concentrate (50% protein at least about 60% denatured) the percent being on a dry solids weight basis, and 4. a whey protein concentrate (50% protein) having a lowered thermogelation temperature prepared by the process of Serial No. 95,684. The formulation and procedure are set forth below:

TABLE I

SHAMPOO FORMULATION

| INGREDIENT | % |
|---|---|
| Na Lauryl Sulfate (28-30%) | 22.5 |
| $NH_4$ Lauryl Sulfate (26-30%) | 22.5 |
| Whey Product* | 2.0 |
| Ethylene Glycol Monostearate | 2.0 |
| Myristyl Monoethanolamide | 0.5 |
| Sorbic Acid | 0.1 |
| Na Benzoate | 0.1 |
| Na Cl | 0.5 |
| Fragrance | 0.5 |
| Distilled Water to | 100.0 |

PROCEDURE:

1. Dissolve the sodium lauryl sulfate and the ammonium lauryl sulfate in the water and heat to 75-80°C.
2. Stir in the salt (NaCl), sorbic acid, and sodium benzoate.
3. Separately melt the myristyl monoethanolamide and the ethylene glycol monostearate. Add these together and heat to 75-80°C.
4. With rapid stirring, slowly add the fatty phase of Step 3 to the blend of Step 2 and stir for 15-20 minutes.
5. With medium stirring, cool the blend to 50°C., and add the whey product followed by addition of the fragrance. Continue cooling but maintain temperature at least about 45°C. for one hour.
6. Cool to room temperature.

The above prepared shampoos showed good pearlescence and were thicker than the control.

EXAMPLE 2

Shampoo formulations containing the whey products identified as 1., 2., 3., and 4. in Example 1 were prepared by dissolving 45% by weight based on the weight of the shampoo ammonium lauryl sulfate (26-30% active ingredient) in 54.2% by weight water and heating to 75-80°C. With medium stirring, the above mixture was cooled to about 50°C. and 0.8% by weight of a whey product was added. The shampoo was cooled to room temperature.

These shampoos were tested against control shampoos without any whey product as follows:

The shampoos were tested according to the following procedure:

1. A 2 gram tress of virgin hair was combed to remove tangles.
2. The tress was washed by wetting the tress under running distilled water until saturated and the excess water removed by running the fingers down the tress.
3. The tress was shampooed by spreading about 20% by weight of the tress of the shampoo down the length of the tress using a syringe, lather for 45 seconds and allow the tress to set for 15 seconds.
4. The tress was rinsed under running distilled water for 30 seconds.
5. The tress was reshampooed according to the procedure of Step 3 using 10% by weight of the tress of shampoo.
6. The tress was rinsed under running distilled water for 30 seconds; and

7. Excess water was blotted from the tress and the tress was allowed to dry in the air.

Strands of hair were examined under a scanning electron microscope at 2,000 and for some samples under 5,000 times magnification. Scanning electron micrographs (SEM) of washed hair (Steps 1., 2. and 7.) show cuticles with sharp, uncoated edges. An SEM of a hair strand shampooed (Steps 1-7) without any whey product showed cuticles with sharp edges similar to the control. An SEM of a strand of hair shampooed (Steps 1-7) with a shampoo containing whey product No. 2. showed an increase in thickness and rounding of the edges of the cuticles. Many cuticles were curled and fiberlike. The presence of a surface coating was also noted indicating substantivity.

An SEM of a hair strand shampooed (Steps 1-7) with a shampoo containing whey product No. 1. showed a softening of the sharp edge of the cuticles. Under 5,000 times magnification, it could be clearly seen that the cuticles were coated with materials as compared to the control with definite rounding of the edges of the cuticles.

An SEM of a hair strand shampooed (Steps 1-7) with a shampoo containing whey product No. 3., showed under 2,000 and 5,000 times magnification a thickening and curling of the cuticles. In addition, the edges of the cuticles were less sharp than the control. Clear evidence of a deposit was noted when compared to the control indicating that whey product No. 3. has a substantive effect on hair.

The previous samples were also prepared omitting rinse Step 6. The SEM's of these strands of hair showed a greater degree of substantivity than those prepared with the final rinse. Good substantivity as compared to a control prepared without a whey product was also shown.

A subjective evaluation showed that samples of hair shampooed by the controls, either rinsed or non-rinsed, were very tatty and hard to comb, fly-away and dull. Hair shampooed with products of the invention, either rinsed or non-rinsed, showed good combability, a decrease in the fly-away character of the hair and were shiny as compared to the control. The whey product identified as No. 3., had better combability than the control but not as good as the combability from the shampoo containing whey product No. 1.

## EXAMPLE 3

Hair conditioners were prepared using the following whey products*, i.e., Product No. 4. of Example 1 and Whey Product No. 5., the precipitate prepared by neutralizing acid whey according to U.S. Patent No. 4,036,999, using the following formulation and procedure:

TABLE II

HAIR CONDITIONER FORMULATION

| | INGREDIENTS | % |
|---|---|---|
| A | Mineral Oil | 35.0 |
| | Microcrystalline Wax | 5.0 |
| | ARLACEL[1] 60 (Sorbitan Monostearate) | 2.0 |
| | TWEEN[1] 60 (Polyoxyethylene 20 Sorbitan Monostearate) | 3.0 |
| B | Distilled Water | 52.7 |
| | Methylparaben | 0.1 |
| C | Whey Product* | 2.0 |
| | Fragrance | 0.2 |
| | | 100.0 |

[1] I.C.I. Americas, Inc.

PROCEDURE:

1. Heat the mineral oil, microcrystalline wax, ARLACEL 60 and TWEEN 60 to 70°C. and stir until clear.
2. Heat the water to 72°C. and with rapid stirring, introduce the methylparaben.
3. With rapid stirring, add the aqueous phase of Step 2 to the oil phase of Step 1 and stir for 30 minutes.
4. Cool the blend to 50°C. and add the whey product followed by the addition of the fragrance.
5. Cool the blend to room temperature while slowly agitating.

The hair conditioners of the invention were pearlescent, superior in visual appearance and viscosity as compared to a control not containing a whey product.

## EXAMPLE 4

Cream rinses were prepared using the whey products* identified as Nos. 1. and 2. in Example 1 and No. 5. in Example 3 using the following formulation and procedure:

### TABLE III
### CREAM RINSE FORMULATION

| INGREDIENTS | % |
|---|---|
| Stearyl Ammonium Chloride | 7.5 |
| Cetyl Alcohol | 0.3 |
| Whey Product* | 2.0 |
| Na Cl | 0.8 |
| Fragrance | 0.3 |
| Distilled Water | 89.1 |
| | 100.0 |

PROCEDURE:

1. Heat the stearyl ammonium chloride in water to 85-87°C.
2. Add the NaCl.
3. Melt the cetyl alcohol and heat it to 87°C.
4. With rapid stirring, slowly add the cetyl alcohol to blend of Step 2 and stir for 15-20 minutes.
5. With slower agitation, cool to 50°C. and add the whey product followed by addition of the fragrance.
6. While stirring slowly, cool to room temperature.

The pH of the cream rinse was adjusted from pH 6.11 to pH 5.5 with the consequence that the cream rinses were somewhat thicker with those containing whey product No. 5. being most acceptable. The cream rinses were pearlescent, superior in visual appearance and viscosity as compared to a control not containing a whey product.

EXAMPLE 5

Skin lotion formulations were prepared with the whey products identified as Nos. 1.-4. in Examples 1 and 5 in Example 3 using the following formulation and procedure:

TABLE IV

SKIN LOTION FORMULATION

| INGREDIENTS | | % |
|---|---|---|
| A | VEEGUM[2] (Magnesium Aluminum Silicate) | 2.0 |
| | Methylparaben | 0.1 |
| | Distilled Water | 66.7 |
| B | Glycerin | 4.0 |
| | Mineral Oil | 8.0 |
| C | LANTROL[3] (Lanolin) | 7.5 |
| | Isopropyl Myristate | 2.0 |
| | Glycerol Monostearate | 2.0 |
| | Cetyl Alcohol | 0.5 |
| | ARLACEL[1] 80 (Sorbitan Monostearate | 1.4 |
| | TWEEN[1] 80 (Polyoxyethylene 20 Sorbitan Monostearate) | 3.6 |
| D | Whey Product* | 2.0 |
| | Fragrance | 0.2 |
| | | 100.0 |

[1] I.C.I. Americas, Inc.

[2] R. T. Vanderbilt

[3] Emery Industries

<u>PROCEDURE</u>:

1. Slowly add the VEEGUM to the water and agitate continuously until smooth. Then add the methylparaben.
2. Add the glycerin and mineral oil to the aqueous phase of Step 1 and heat to 70°C.
3. Blend the ingredients identified in Section <u>C</u> and heat to 75°C. Then add this heated blend to the blend of Step 2 with rapid mixing.
4. Cool the blend of Step 3 to 50°C. with slower mixing and then add the whey product, followed by addition of the fragrance.
5. Cool the entire blend to room temperature with slow mixing.

The skin lotions were acceptable in visual appearance and viscosity. The skin lotions containing the whey products were less greasy to the touch than a control prepared without a whey product. The whey products No. 4. and 5. provided products which were superior in visual appearance, viscosity and feel as compared to the other whey products and the control.

Similar effects can be obtained in other body lotions such as moisturizing lotion, moisturizing cream, cleansing cream, cleansing lotion and night cream. Appropriate formulations for these and other cosmetics are shown in the article in the September 1973 issue of Drug and Cosmetic Industry entitled "A New Natural Ingredient for Cosmetic Formulators", the contents of which are incorporated herein by reference. The protein used in these formulations is an oat based product having 13% protein, 8% lipid and 77% carbohydrate. These formulations are given as illustrative of various cosmetic formulations in which whey products can be used in accordance with the invention.

CLAIMS:

1. A cosmetic formulation for treating skin or hair comprising said cosmetic in combination with from about 0.5% to about 10% by weight based on the total weight of the formulation a whey protein-containing product which is substantially non-hydrolyzed, said whey product being selected from the group consisting of whey, delactosed whey, demineralized whey, whey protein concentrate, the precipitate obtained by neutralizing a whey product of at least 40% acid whey or a calcium enriched whey, the material remaining after removal of said precipitate from said whey, the protein-containing byproducts remaining after the preparation of whey protein concentrate and mixtures thereof.

2. The cosmetic formulation as recited in claim 1 wherein said whey product is selected from the group consisting of whey protein concentrate, said precipitate, said byproducts and mixtures thereof.

3. The cosmetic formulation as recited in claim 1 wherein said whey protein concentrate is characterized by a lowered gelation temperature and is capable of forming a gel at 15% solids in water at 75°C. within 30 minutes, said lowered gelation temperature whey protein concentrate being prepared by a process comprising:

a) adjusting the pH of an aqueous solution of a whey protein concentrate having a temperature of less than about 30°C. to a pH within the range of from about 8 to about 10, the total dissolved protein content being less than about 15% when determined at said pH;

b) heating the alkaline solution of step a) to an elevated temperature within the range of from about 50°C. and about 80°C.;

c) cooling said heated solution to a temperature within the range of from about 30°C. to about 2°C. within 30 minutes after the said solution reaches its maximum temperature level, said cooling being conducted at a rate sufficient to prevent gelation of the whey protein containing solution.

4. The cosmetic formulation as recited in claim 1 wherein said whey product is a blend of from about 25% to about 75% of said precipitate from an acid whey blend with the remainder being from about 75% to about 25% of a whey protein concentrate in which the whey protein is substantially denatured.

5. The cosmetic formulation as recited in claims 1, 3 and 4 wherein the protein content of said whey protein concentrate ranges from about 40% to about 60%.

6. The cosmetic formulation as recited in claim 1 wherein said cosmetic is selected from the group consisting of shampoo, hair groom, hair cream rinse, hair conditioner, hair setting lotion, permanent wave, hair straighteners, hair coloring, hair lighteners, skin cleansing formulations, dusting powder, emollient formulations, skin therapeutic formulations, skin softening formulations, sun tanning and screening formulations, makeup foundation, makeup, beauty masks, shaving preparations and depilatories.

7. The cosmetic formulation as recited in claim 6 wherein said cosmetic is selected from the group consisting of shampoo, hair conditioner, hair cream rinse and skin cleansing formulations.

8. The cosmetic formulation as recited in claim 1 wherein said whey product is present in an amount ranging from about 0.75% to about 5%.

9. The cosmetic formulation as recited in claim 1 wherein said whey product is present in an amount ranging from about 0.75% to about 3%.

10. A process for preparing a cosmetic formulation for treating skin or hair comprising adding to said cosmetic during its manufacture from about 0.5% to about 10% by weight based on the total weight of the formulation of a whey protein-containing product which is substantially non-hydrolyzed, said whey product being selected from the group consisting of whey, delactosed whey, demineralized whey, whey protein concentrate, the precipitate obtained by neutralizing a whey product of at least 40% acid whey or a calcium enriched whey, the material remaining after removal of said precipitate from said whey and the protein-containing byproducts remaining after the preparation of whey protein concentrate and mixtures thereof.

11. The process as recited in claim 10 wherein said whey product is selected from the group consisting of whey protein concentrate, said precipitate, said byproducts and mixtures thereof.

12. The process as recited in claim 10 wherein said whey protein concentrate is characterized by a lowered gelation temperature and is capable of forming a gel at 15% solids in water at 75°C. within 30 minutes, said lowered gelation temperature whey protein concentrate being prepared by a process comprising:

a) adjusting the pH of an aqueous solution of a whey protein concentrate having a temperature of less than about 30°C. to a pH within the range of from about 8 to about 10, the total dissolved protein content being less than about 15% when determined at said pH;

b) heating the alkaline solution of step a) to an elevated temperature within the range of from about 50°C. and about 80°C.;

c) cooling said heated solution to a temperatue within the range of from about 30°C. to about 2°C. within 30 minutes after the said solution reaches its maximum temperature level, said cooling being conducted at a rate sufficient to prevent gelation of the whey protein containing solution.

13. The process as recited in claim 10 wherein said whey product is a blend of from about 25% to about 75% of said precipitate from an at least 40% acid whey blend with the remainder being from about 75% to about 25% of a whey protein concentrate in which the whey protein is substantially denatured.

14. The process as recited in claims 10, 12 and 13 wherein the protein content of said whey protein concentrate ranges from about 40% to about 60%.

15. The process as recited in claim 10 wherein said cosmetic is selected from the group consisting of shampoo, hair groom, hair cream rinse, hair conditioner, hair setting lotion, permanent wave, hair straightener,hair coloring, hair lighteners, skin cleansing formulations, dusting powder, emollient formulations, skin therapeutic formulations, skin softening formulations, sun tanning and screening formulations, makeup foundation, makeup, beauty masks, shaving preparations and depilatories.

16. The process as recited in claim 15 wherein said cosmetic is selected from the group consisting of shampoo, hair conditioner, hair cream rinse and skin cleansing formulations.

17. The process as recited in claim 10 wherein said whey product is present in an amount ranging from about 0.75% to about 5%.

18. The process as recited in claim 10 wherein said whey product is present in an amount ranging from about 0.75% to about 3%.

19. A method for setting hair which comprises applying to said hair a non-hydrolyzed whey product in an amount sufficient to set the hair upon application of temperature and applying a temperature at a level sufficient to set said whey product to thereby set hair.